# EUROPEAN PATENT APPLICATION

(11) **EP 2 740 800 A1**
(43) Date of publication of application: **11.06.2014**
(21) Application number: 12195471.3
(22) Date of filing: 04.12.2012
(51) Int. Cl.: C12Q 1/00

(54) **Cellulase assay**

(71) Applicant: Megazyme IP Limited, Bray Wicklow (IE)
(72) Inventor: McCleary, Barry Vincent, Co. Wicklow (IE); Mangan, David Peter, Co. Dublin (IE)
(74) Representative: Gates, Marie Christina Esther

(57) **Abstract**

The invention involves the development of a substrate and assay procedure for the scientific measurement of cellulase in admixture with other enzymes active on cellulose or 1,4-β-D-cello-oligosaccharides, or in various levels of purity of the cellulase. This substrate being characterised by the fact that it contains at least 2 glucose units linked β-1,4-, with the reducing end glucose unit linked β- or α- to a compound which exhibits an optically measurable change on cleavage of the bond between it and the adjacent D-glucosyl residue and the non-reducing terminal D-glucosyl unit is covalently linked to a blocking substituent which inhibits cleavage by exo-acting enzymes of the bond between the terminal and non-reducing end, penultimate D-glucose unit.

## Description

### Field of the Invention

The invention relates to methods for the specific measurement of *endo-*1,4-β-D-glucanase (hereafter called cellulase) and to assay kits for such measurement. Such methods and kits find use in the measurement of cellulase in the analysis of microbial fermentation products, in industrial enzyme preparations and other biological materials containing cellulase enzyme activity and in standardising cellulase for biofuel production.

### Back2round to the Invention

Numerous assays have been described for the specific and non-specific measurement of cellulase in biological materials including those based on the measurement of increase in reducing sugars on hydrolysis of cello-oligosaccharides, chemically modified or non-modified cellulose or 1,3;1,4-β-D-glucan (McCleary, *et al.,* 2012); increase in dyed fragments soluble in an aqueous/organic solvent on hydrolysis of soluble dyed polysaccharides; increase in water soluble, dyed fragments or hydrolysis of insoluble, dyed and crosslinked polysaccharides (McCleary, *et al.,* 2012). Japanese Patent JP02083390 describes the preparation and use of nitrophenyl-cello-oligosacchardes for the measurement of cellulase, and such nitrophenyl-cello-oligosaccharides have been used by Rahman *et al.* (2002) in studies on the action pattern of *Thermatoga maritima endo-*1,4-β-glucanase (cellulase). However, since cellulase occurs in admixture with other enzymes which are active on nitrophenyl-cello-oligosaccharides, namely, β-glucosidase and *exo-*1,4-β-D-glucanases, the described substrates cannot be used for the specific and quantitative measurement of cellulase in fermentation broths and industrial enzyme preparations.

Blair (1988) describes a procedure for the measurement of α-amylase in biological fluids using end-blocked nitrophenyl malto-oligosaccharides. However, since the date of that invention, no parallel substrate or assay procedure has been developed or published for measurement of cellulase, although such a compound or product would be highly desirable.

### Object of the Invention

It is thus an object of the present invention to provide methods, reagents and kits for the specific determination and measurement of cellulase, particularly, but not exclusively, where it occurs in admixture with other enzymes which are active on nitrophenyl-cello-oligosaccharides, such as β-glucosidase and *exo-*1,4-β-D-glucanases. A further object is to provide methods, reagents and kits which can be used for the specific and quantitative measurement of cellulase in fermentation broths and industrial enzyme preparations and the like. A further object of the invention is to provide methods, reagents and kits for cellulase measurement and determination, which can be produced commercially, are easy to conduct and are reliable.

### Summary of the Invention

According to the present invention there are provided reagent kits for measurement of cellulase comprising, an oligosaccharide substrate for assay of cellulase, the said substrate containing at least 2 glucose units linked by a 1,4- β-linkage, with the reducing-end glucose of the oligosaccharide linked via a bond cleavable by β- or α-glucosidase, to a label which exhibits a measurable change upon cleavage of the bond, and the non-reducing glucose unit being linked to a chemical blocking substituent which inhibits cleavage by *exo*-enzymes of the bond between the non-reducing end glucose unit and the adjacent glucose unit.

The kit may further comprise an *exo*-enzyme capable of cleaving the bonds between the non-blocked glucose residues and the label. This enzyme may be β-glucosidase.

The kit may further comprise a second exo-enzyme if the oligosaccharide is α-linked to the label. This enzyme may be α-glucosidase.

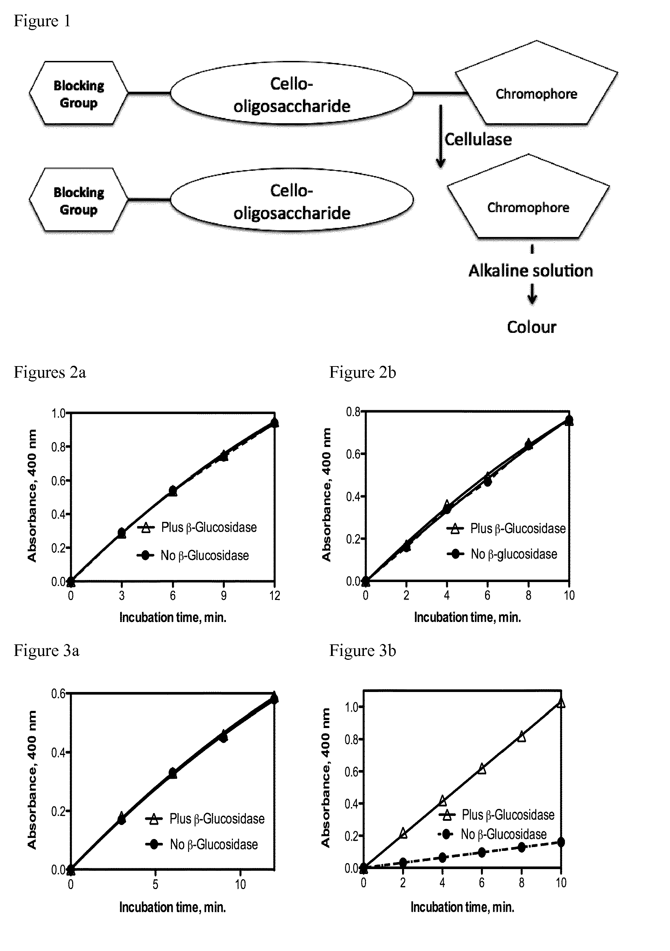

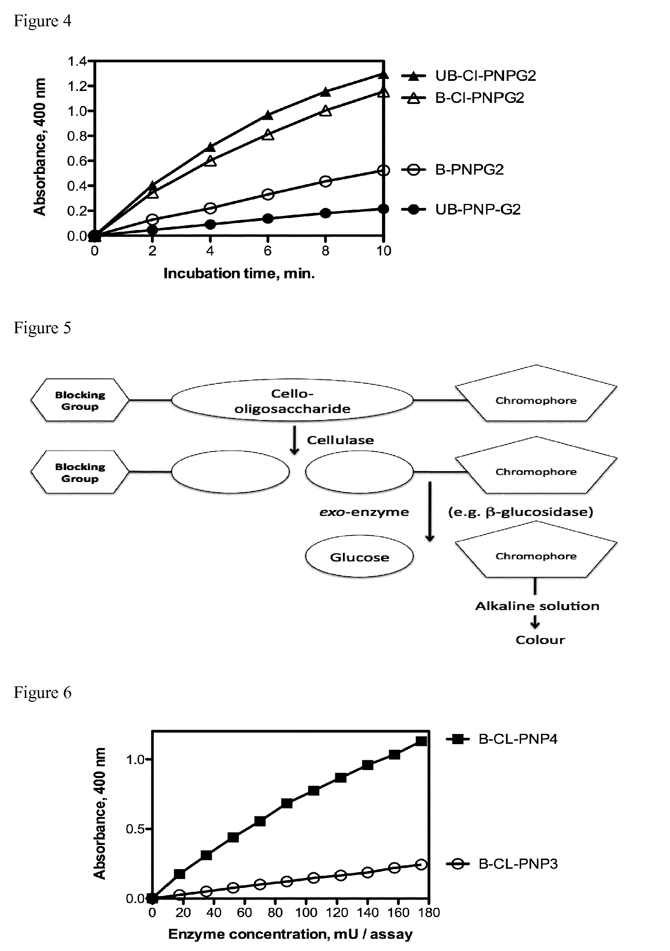

There are thus two embodiments of the present invention, depending on the application. In the first embodiment of this invention oligosaccharides composed of two or three D-glucosyl residues, can be hydrolysed by certain cellulases with direct release of the label e.g. 2-chloro-4-nitrophenol (ClPNP) from the end-blocked cello-oligosaccharide (as shown in Figure 1). The second embodiment of this invention involves the use of the same end-blocked ClPNP-β-cello-oligosaccharides containing three or more D-glucosyl residues together with an *exo*-enzyme, such as β-glucosidase, capable of cleaving the bonds between the non-blocked glucose residues and the label (Figures 5-8).

This invention includes the steps involved in modifying the labelled cello-oligosaccharides to provide end-blocked colourimetric/fluorimetric substrates for cellulase as well as methods for dissolution of the end-blocked nitrophenyl-cello-oligosaccharides and optimised ancilliary enzyme(s), where incorporated, for use with the oligosaccharide subtrates.

In general, this invention involves the development of substrates and assay procedures for the scientific measurement of cellulase in admixture with other enzymes active on cellulose or 1,4-β-D-cello-oligosaccharides, or in various levels of purity of the cellulase. This substrate is characterised by the fact that it contains at least 2 glucose units linked β-1,4-, with the reducing end glucose unit linked β- or α- to a compound which exhibits an optically measurable change on cleavage of the bond between it and the adjacent D-glucosyl residue. Furthermore, and critically, the non-reducing terminal D-glucosyl unit is covalently linked to a blocking substituent, which inhibits cleavage by *exo*-acting enzymes of the bond between the terminal and non-reducing end, penultimate D-glucose unit. This blocking group itself, not being susceptible to hydrolysis by enzymes in the mixture being analysed, e.g. esterase or lipase enzymes. The invention also embodies a method for solubilising said end-blocked, nitrophenyl cello-oligosaccharide allowing its use in enzyme assay procedures.

In the first embodiment, reaction is initiated by mixing solubilised substrate and the *endo*-acting cellulase. Reaction is followed automatically or manually depending on the nature of the label used and the pH of the reaction mixture.

In the second embodiment, reaction is initiated by mixing solubilised substrate, *exo*-enzyme capable of hydrolysing the link between the label and α- or β-linked glucose, and the *endo*-acting cellulase (Figure 5). Reaction is followed automatically or manually depending on the nature of the label used and the pH of the reaction mixture.

In preferred embodiments, the label is a chromophore, a fluorophore, a chemiluminescent substituent, or a bioluminescent substituent; the *exo*-enzyme, where included, is β-glucosidase, but may also be a mixture of β-glucosidase and α-glucosidase. Preferred labels include 2-Cl-4-nitrophenol (ClPNP), *p*-nitrophenol (PNP), o-nitrophenol (chromophores), coumarin derivatives such as 4-methylumbelliferone (a fluorophore), and luciferin (a chemiluminescent substituent). Preferably, the substrate has six or fewer glucose units, and most preferably has two, three or four. Preferred blocking substituents are acetals or ketals, e.g. benzylidene. The reagents used in the assay are preferably provided in the form of a reagent kit, the reagents preferably being mixed at the time of assay. The assays of the invention work according to the general schemes shown in Figures 1 and 5. In the scheme illustrated in Figure 1 (First embodiment), the blocking group prevents *exo*-enzymes from breaking down the substrate, so that in the absence of cellulase there will be no colour change. Cellulase, an *endo*-enzyme, cleaves the linkage between the reducing end terminal D-glucosyl residue and the 2-chloro-4-nitrophenyl group in the substrate, causing the direct release of the chromophore.

In the scheme illustrated in Figure 5 (second embodiment), the blocking group again prevents *exo*-enzymes from breaking down the substrate, so that in the absence of cellulase there will be no colour change. In this case, cellulase, an *endo*-enzyme, cleaves internal β-1,4 bonds in the substrate, producing smaller fragments which can be acted on by the *exo*-enzyme, β-glucosidase, which causes the ultimate release of the chromophore.

The invention also provides a process for the dissolution of the end-blocked *p-*nitrophenyl cello-oligosaccharide or end-blocked 2-Cl-4-nitrophenyl cello-oligosaccharide, either of which is sparingly soluble in water or buffer solutions, comprising use of a chaotropic solvent. Preferably, the co-solvent inhibits neither the cellulase being measured nor the enzymes β-glucosidase and α-glucosidase if they are incorporated into the assay. An example of a suitable solvent is dimethyl sulphoxide.

The assay of the invention produces a linear, or near-linear result, i.e. optical density (400 nm) measurements are proportional to cellulase concentration and/or the time of incubation, rendering the assay highly amenable to automation. In addition, the components of the assay (substrate dissolved in dimethyl sulphoxide and potassium chloride solution and β-glucosidase) can be combined before use and show excellent stability over several hours at room temperature, because the blocking group prevents degradation of the substrate by β-glucosidase.

### Detailed Description of the Drawings

**Figure 1****.** is a diagrammatic representation of the assay scheme of the invention in accordance with the first embodiment. The *endo*-acting celluase cleaves the glycosidic bond between the terminal D-glucosyl unit and ClPNP directly releasing ClPNP.
**Figure 2****.** is a graph of absorbance (400 nm) vs. time, for the action of a cellulase from *Aspergillus niger* on; 2a) end-blocked 2-chloro-4-nitrophenyl cellobiose (BClPNP-G2) (17500 milli-International Units per assay) and; 2b) end-blocked 2-chloro-4-nitrophenyl cellotriose (BClPNP-G3) (700 milli-International Units per assay) at 40°C and pH 4.5 in the presence and the absence of β-glucosidase using an assay of the invention. With both BClPNP-G2 and BClPNP-G3, the *Aspergillus* cellulase cleaves the glycosidic bond between the terminal glucosyl unit and the ClPNP with immediate release of blocked cello-oligosaccharide and free ClPNP, as shown by the same sensitivity of the assay (rate of release of ClPNP) in the presence or absence of β-glucosidase. **Note:** One International Unit of enzyme activity as defined is the amount of enzyme required to release one micromole of reducing sugar equivalents per minute from carboxymethyl cellulose 4M (CMC-4M) (10 mg/mL) in 100 mM sodium acetate buffer (pH 4.5).
**Figure 3****.** is a graph of absorbance (400 nm) vs. time, for the action of a cellulase from *Trichoderma longibrachiatum* on; 3a) BClPNP-G2 (600 milli-International Units per assay) and on; 3b) BClPNP-G3 (30 milli-International Units per assay) at 40°C and pH 4.5 in the presence and the absence of β-glucosidase using an assay of the invention. With BClPNP-G2 (3a), *Trichoderma* cellulase cleaves the glycosidic bond between the terminal glucosyl unit and the ClPNP with immediate release of end-blocked cello-oligosaccharide and free ClPNP as shown by the same sensitivity of the assay (rate of release of ClPNP) in the presence or absence of β-glucosidase. In contrast, for BClPNP-G3 (3b) the *endo*-acting *Trichoderma* cellulase cleaves the glycosidic bond either between two glucosyl units or between the bond between the terminal glucosyl unit and ClPNP releasing an end-blocked cello-oligosaccharide and a non-blocked ClPNP-cello-oligosaccharide fragment, or free ClPNP. The β-glucosidase in the assay mixture immediately hydrolyses the non-blocked ClPNP-cello-oligosaccharide released, giving D-glucose and free ClPNP. This is shown by the greater sensitivity of the assay in the presence of added β-glucosidase than in the absence of this enzyme.
**Figure 4****.** is a graph of absorbance (400 nm) vs. time, for the action of a cellulase from *Trichoderma longibrachiatum* (1500 milli-International Units on CMC-4M per assay) on *p*-nitrophenyl cellobiose (PNP-G2), 2-chloro-4-nitrophenyl cellobiose (ClPNP-G2), end-blocked *p*-nitrophenyl cellobiose (BPNP-G2) and end-blocked 2-chloro-4-nitrophenyl cellobiose (BClPNP-G2) in the absence of added β-glucosidase at 40°C and pH 4.5 using an assay of the invention (embodiment Two).
**Figure 5**. is a diagrammatic representation of the assay scheme of the invention as per *Format Two.* The *endo*-acting celluase cleaves the glycosidic bond between two glucosyl units and/or the bond between the terminal glucosyl unit and ClPNP releasing an end-blocked cello-oligosaccharide and an unblocked ClPNP-cello-oligosaccharide fragment, or free ClPNP. The β-glucosidase (and/or α-glucosidase) in the assay mixture immediately hydrolyses the unblocked ClPNP-cello-oligosaccharide released, giving glucose and free ClPNP.
**Figure 6****.** is a graph of absorbance (400 nm) vs. enzyme concentration, for the action of a cellulase from *Aspergillus niger* (17.5 to 175 milli-International Units on CMC-4M per assay) on BClPNP-G3 and BClPNP-G4 in the presence of β-glucosidase at 40°C and pH 4.5 for 10 min, using an assay of the invention (embodiment Two).

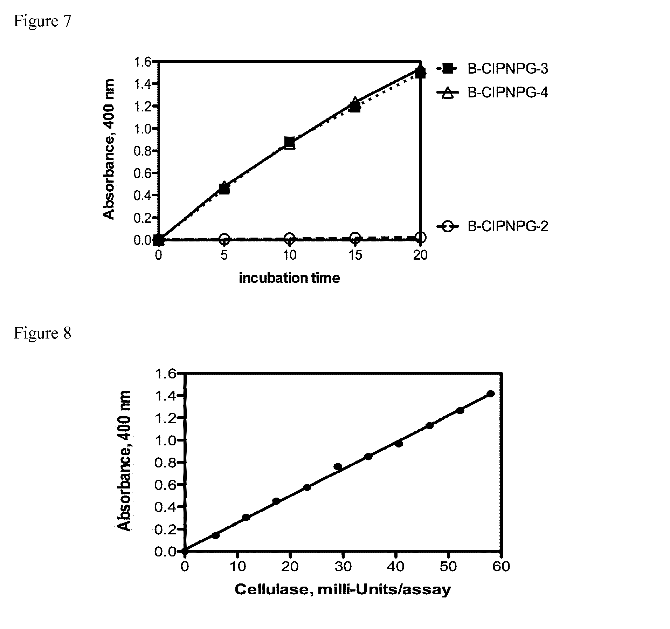

**Figure 7****.** is a graph of absorbance (400 nm) vs. time, for the action of *Trichoderma longibrachiatum* cellulase (30 milli-International Units on CMC-4M per assay) on BClPNP-G2, BClPNP-G3 and BClPNP-G4 in the presence of β-glucosidase at 40°C and pH 4.5 using an assay of the invention (embodiment Two).
**Figure 8****.** is a graph of absorbance (400 nm) vs. enzyme concentration, for the action of a cellulase from *Thermatoga maritima* (35 to 350 milli-International Units on CMC-4M at 80°C per assay) on BClPNP-G4 in the presence of β-glucosidase at 80°C and pH 6.5 for 10 min, using an assay of the invention (embodiment Two).

### STRUCTURE AND SYNTHESIS OF SUBSTRATE

The oligosaccharide portion of the substrate can generally be obtained commercially, or can be produced by acid or enzymic hydrolysis of cellulose or cellulose derivatives, as is known to those skilled in the art.

The label portion, preferably a chromophore such as 2-chloro-4-nitrophenol, *p-*nitrophenol or o-nitrophenol, or a fluorophore such as 4-methylumbelliferone, can be attached to the reducing-end glucose unit using standard techniques, e.g. those described in Planas *et al.* (1998) and Dess *et al.* (1981) and cellobiose labelled with 2-chloro-4-nitrophenol is commercially available, e.g. from Megazyme or Carbosynth.

The blocking group can be any substituent which prevents *exo*-enzymes from breaking down the substrate. The blocking group works by creating a terminal glucose unit no longer capable of fitting the active site of the *exo*-enzyme. Thus, the size and chemical composition of the blocking group are not critical; all that is required is that interaction between the enzyme and the substrate is prevented. Any substituent bonded to C2, C3, C4 or C6 of the terminal glucose unit that blocks the action of *exo*-enzymes can be used.

One class of blocking groups can replace a hydrogen in the hydroxyl group of C6 of the terminal glucose unit. Suitable such groups include, e.g. carboxylic acid esters (e.g. acetyl or benzoyl); phosphate esters; sulfonate esters (e.g. toluenesulfonyl or methanesulfonyl); ethers (e.g. benzyl, silyl and triphenylmethyl).

Alternatively, the blocking group can be an acetal or ketal functional group, i.e. a group which blocks the C4 and C6 hydroxyls of the terminal glucose unit.

Synthesis of a blocked substrate for use in the assay of the invention can be carried out according to the following general scheme; the illustrated chromophore is either *p*-nitrophenol or 2-Cl-4-nitrophenol, the blocking group is benzylidene, and there are n + 1 glucose units, where 1 ≤ n ≤ 5:

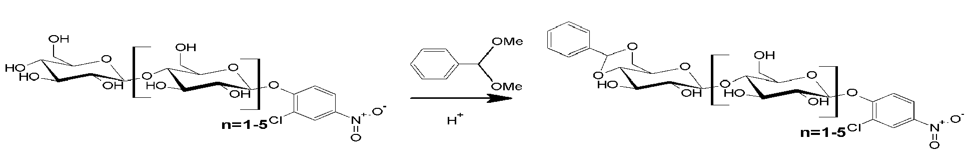

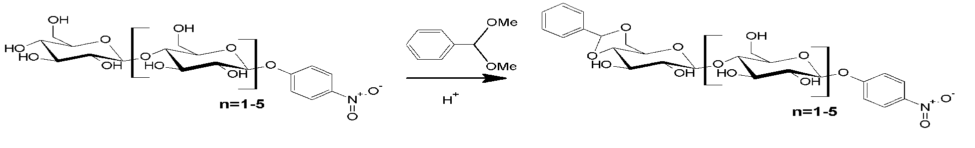

A substrate containing four glucose units, blocked with benzylidene and labelled with 2 chloro-4-nitrophenol, has the formula O-(4,6-O-benzylidene-β-D-glucopyranosyl)-(-4)-O-(β-D-glucopyranosyl)-(1-4)-O-(β-D-glucopyranosyl)-1-O-(2-chloro-4-nitrophenol-O-β-D-glucopyranoside) **(1),** and the structure:

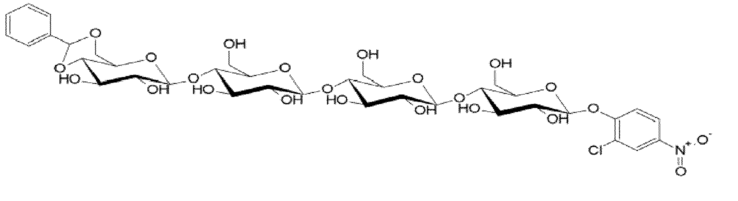

Compound **(1)** was synthesised as follows: To a solution of 2-Cl-4-nitrophenol cellotetraoside (1 g, 1.217 mmol) and *p*-toluenesulfonic acid monohydrate (69 mg, 0.365 mmol) in anhydrous dimethylformamide (30 mL) under an argon atmosphere containing activated 4A molecular sieves (200 mg) was added benzaldehyde dimethylacetal (917 µL, 6.083 mmol). The reaction was heated to 50°C and stirred for 14 hours. After this time, TLC analysis indicated reaction completion. Triethylamine (69 µL, 0.494 mmol) was added and the reaction cooled to room temperature. The crude reaction mixture was absorbed onto silica gel and semi-purified by flash chromatography. The fractions obtained containing the desired product were combined and further purified by recrystallization from a 2: 1 mixture of acetonitrile and water.

The product of this reaction had the formula O-(4,6-O-benzylidene-β-D-glucopyranosyl)-(1-4)-O-(β-D-glucopyranosyl)-(1-4)-O-(β-D-glucopyranosyl)-1-O-(2-chloro-4-nitrophenol-O-β-D-glucopyranoside) **(1).** The desired product was obtained as a white solid (390 mg, 0.429 mmol, 35%).

In a first embodiment oligosaccharides composed of two or three D-glucosyl residues, can be hydrolysed by certain cellulases with direct release of the label e.g. 2-chloro-4-nitrophenol (ClPNP) from the end-blocked cello-oligosaccharide (as shown in Figure 1). Examples of this are shown in Figures 2a and 2b where it can be seen that the inclusion of the ancilliary enzyme, β-glucosidase, gives no increase in the colour formed (rate of release of ClPNP) on reaction of *Aspergillus niger* cellulase with end-blocked ClPNP-β-cellobiose (BClPNP-G2) or end-blocked ClPNP-β-cellotriose (BClPNP-G3). In Figure 3a, the same result is seen on incubation of *Trichoderma longibrachiatum* cellulase with BClPNP-G2. From the results in Figure 4, it is evident that while end-blocked p-nitrophenyl cellobiose (BPNP-G2) is also hydrolysed by *Trichoderma* cellulase, this is at a slower rate than BClPNP-G2. The blocked substrates are hydrolysed at a rate similar to that of the unblocked substrates, but the latter cannot be used to assay cellulase activity in preparations containing β-glucosidase or *exo-*1,4-β-glucanase.

### CELLULASE (endo-1,4-β-GLUCANASE) ASSAY

### Substrate Dissolution

Prepare substrate solution by dissolving end-blocked *p*-nitrophenyl cello-oligosaccharides or end-blocked 2-Cl-4-nitrophenyl cello-oligosaccharide (DP 2, 3 or 4) in 2 mL of dimethyl sulphoxide to obtain a concentration of 12.5 mM. Cool this solution in ice and add 3 mL of 170 mM potassium chloride solution with mixing, to give a final substrate concentration of 5 mM. Prepare this solution fresh before use, or warm to 40°C before use to ensure complete dissolution.

### Manual Assay Format

Add an aliquot (0.1 mL) of the substrate solution to the bottom of a 16 x 120 mm glass test tube, followed by 0.05 mL of pure β-glucosidase (Second embodiment only). Incubate tubes for 2 min at the selected reaction temperature and initiate the reaction by adding 0.1 mL of sample solution containing cellulase in 200 mM buffer solution of the desired pH (usually in the range of 4.5 to 8.0). After incubation for the desired time (e.g. 10 min), terminate the reaction by adding 3 mL of 2 % w/v Tris buffer (pH 9) or tri-sodium phosphate solution (pH 11). Mix the tube contents and measure the absorbance at 400 nm against a reaction blank.

### Microplate Assay Format

Add an aliquot (0.02 mL) of the substrate solution to the bottom of the micro-well followed by 0.02 mL of pure β-glucosidase (Second embodiment only). Mix well, and after 1 min at 40°C, initiate the reaction by adding 0.02 mL of sample solution containing cellulase in 200 mM buffer solution of the desired pH (usually in the range of 4.5 to 8.0). After incubation for the desired time (e.g. 10 min), terminate the reaction by adding 0.3 mL of 2 % w/v tris buffer (pH 9) or tri-sodium phosphate solution (pH 11). Mix the tube contents and measure the absorbance at 400 nm against a reaction blank. Run standard cellulase preparations concurrently.

### REAGENT KIT

The reagents used in the assay method of this invention are preferably provided in the form of a reagent kit which includes:
1. Blocked, labelled substrate (preferably containing 3, 4, or 5 glucose units) (either as a powder or as a stabilised solution ready for use).
2. Pure β-glucosidase (either as a stabilised solution or lyophilised powder).
3. A cellulase control preparation (either as a stabilised solution or lyophilised powder).

### REFERENCES

Planas, A., Abel, M., Millet, J., Palasi, Ó., Pallarés, C. and Viladot, J.L. Synthesis of aryl 3-O-β-cellobiosyl-β-D-glucopyranosides for reactivity studies of 1,3-1,4-β-glucanases. Carbohydrate Research (1998) 310:53-64.
Dess, D., Kleine, H.P., Weinberg, D.V., Kaufman, R.J. and Sidhu, R.S. Phase-transfer catalysed synthesis of acetylated aryl β-D-glucopyranosides and aryl β-D-galactopyranosides. Synthesis (1981) 883-885.
McCleary, B.V., McKie, V. and Draga, A. Measurement of 1,4-β-glucanase. Methods in Enzymology, (2012) 510:1-17.
Rahman, Md.M, Bhuiyan, S.H., Nirasawa, S, Kitaoka, M and Hayashi, K. Characterisation of an endo-β-1,4-glucanase of Thermatoga maritima expressed in Escherichia coli. J. Appl. Glycoscience (2002) 49:487-495.

The words "comprises/comprising" and the words "having/including" when used herein with reference to the present invention are used to specify the presence of stated features, integers, steps or components but does not preclude the presence or addition of one or more other features, integers, steps, components or groups thereof. It is appreciated that certain features of the invention, which are, for clarity, described in the context of separate embodiments, may also be provided in combination in a single embodiment. Conversely, various features of the invention which are, for brevity, described in the context of a single embodiment, may also be provided separately or in any suitable sub-combination.

## Claims

1. A reagent kit for measurement of cellulase comprising, an oligosaccharide substrate for assay of cellulase, the said substrate containing at least 2 glucose units linked by a 1,4- β-linkage, with the reducing-end glucose of the oligosaccharide linked via a bond cleavable by β- or α-glucosidase, to a label which exhibits a measurable change upon cleavage of the bond, and the non-reducing glucose unit being linked to a chemical blocking substituent which inhibits cleavage by *exo*-enzymes of the bond between the non-reducing end glucose unit and the adjacent glucose unit.

2. A reagent kit as claimed in claim 1 further comprising an *exo*-enzyme capable of cleaving the bonds between the non-blocked glucose residues and the label.

3. A kit as claimed in claim 2 further comprising a second *exo*-enzyme if the oligosaccharide is α-linked to the label.

4. A kit as claimed in claim 1, 2 or 3 wherein the measurable change upon cleavage of the bond is optically measurable.

5. A kit as claimed in any preceding claim wherein the label is a chromophore, a fluorophore, a chemiluminescent substituent, or a bioluminescent substituent.

6. A kit as claimed in claim 5 wherein the label is selected from 2-chloro-4-nitrophenol (ClPNP), *p*-nitrophenol (PNP), o-nitrophenol, coumarin derivatives such as 4-methylumbelliferone, and luciferin.

7. A kit as claimed in any of claims 2 to 6 wherein the first *exo*-enzyme is β-glucosidase or a mixture of β-glucosidase and α-glucosidase.

8. A kit as claimed in any of claims 3 to 7 wherein the second *exo*-enzyme is α-glucosidase.

9. A kit as claimed in claims 2-8 wherein the substrate has six or fewer glucose units.

10. A kit as claimed in claim 9 wherein the substrate has two, three or four glucose units.

11. A kit as claimed in any preceding claim wherein the blocking substituents are acetals or ketals, e.g. benzylidene.

12. A method for the measurement of cellulase in a sample comprising incubating the sample with an oligosaccharide substrate and with or without an exo-enzyme, the substrate containing at least 2 glucose units linked by a 1,4-β-linkage, with the reducing-end glucose of the oligosaccharide linked via a bond cleavable by β- or α-glucosidase, to a label which exhibits a measurable change upon cleavage of the bond, the exo-enzyme if present being capable of cleaving the bond between the reducing-end glucose unit and the label, and measuring a change between the sample and a control, on cleavage of the bond, the change indicating the amount of cellulase in the sample.

13. A process for the dissolution of the end-blocked *p*-nitrophenyl cello-oligosaccharide or end-blocked 2-Cl-4-nitrophenyl cello-oligosaccharide, either of which are sparingly soluble in water or buffer solutions, comprising use of a chaotropic solvent.

14. A process as claimed in claim 12 wherein the co-solvent used inhibits neither the cellulase being measured nor the enzymes β-glucosidase and α-glucosidase where used in the assay.

15. A process as claimed in claim 13 wherein the solvent is dimethyl sulphoxide or another chaotrophic solvent able to dissolve the substrate.
